# EUROPEAN PATENT APPLICATION

(11) **EP 2 462 866 A2**
(43) Date of publication of application: **13.06.2012**
(21) Application number: 11193375.0
(22) Date of filing: 13.12.2011
(51) Int. Cl.: A61B 5/0245, A61B 5/145

(54) **Heart rate monitor**

(30) Priority: 13.12.2010 US 966864
(71) Applicant: Scosche Industries, Inc., Oxnard, CA 93033 (US)
(72) Inventor: Buchheim, James, Oxnard, CA 93033 (US); Hennig, Arne, Fort Lauderdale, FL 33328 (US)
(74) Representative: Hill, Justin John

(57) **Abstract**

A heart rate monitor includes a user system (100) adjacent to a user's skin in communication with a remote processing system (200). The user system includes a user processor (105), a user memory (110) coupled to the user processor, a clock signal generator (115) coupled to the processor, a sensing system (120) coupled to the processor for measuring at least a user heart rate, a user transceiver (125) coupled to the processor, a user interface (135) coupled to the processor, and a user antenna (145) coupled to the transceiver. A user battery (150) is coupled to the user processor, the user memory, the clock signal generator, the sensing system, and the user transceiver. The remote processing system includes a remote processor (205), a remote memory (210) coupled to the remote processor, a remote transceiver (225) coupled to the remote processor, and a remote antenna (245) coupled to the remote transceiver. The sensing system includes a blood concentration sensing system (310) configured to sense changes in blood concentration in one or more blood vessels beneath a surface of the skin, a motion sensor (320) for sensing changes in position of the heart rate monitor with respect to the skin, and an accelerometer (330) for sensing motion of the heart rate monitor with respect to a user's heart.

## Description

### BACKGROUND

### Field

The present disclosure relates generally to health monitoring systems and methods, and more particularly to monitoring heart rate under various conditions of exercise.

### Background

A pulse is the rate at which the heart beats, measured in beats per minute (bpm). Basil pulse is the pulse measured at rest. The pulse measured during physical activity is generally higher than the basil pulse, and the rise in pulse during physical exertion is a measure of the efficiency of the heart in response to demand for blood supply.

A person engaging in physical activity often wishes to monitor the heart rate via pulse measurement in order to monitor and/or regulate the degree of exertion, depending on whether the exercise is intended for fitness maintenance, weight maintenance/reduction, cardiovascular training, or the like.

A standard method of measuring pulse manually is to apply gentle pressure to the skin where an artery comes close to the surface, e.g., at the wrist, neck, temple area, groin, behind the knee, or top of the foot. However, measuring pulse this way during exercise is usually not feasible. Therefore, numerous devices provide pulse measurement using any of a variety of sensors attached to the body in some fashion. Monitors attached to the wrist, chest, ankle and upper arm, are preferably placed over a near-skin artery, are common. The method of measurement may involve skin contact electrodes.

A wireless heart rate monitor conventionally consists of a chest strap sensor-transmitter and a wristwatch-type receiver. The chest strap sensor has to be worn around the chest during exercise. It has two electrodes, which are in constant contact with the skin, to detect electrical activities coming from the heart. Once the chest strap sensor-transmitter has picked up the heart signals, it transmits the information wirelessly and continuously to the wristwatch. The number of heart beats per minute is then calculated and the value displayed on the wristwatch.

The wireless heart rate monitor can be further subdivided into digital and analog, depending on the wireless technology the chest strap sensor-transmitter uses to transmit information to the wristwatch. The wireless heart rate monitor with analog chest strap sensor-transmitter is a popular type of heart rate monitors. There is, however, a possibility of signal interference (cross-talk) if other analogue heart rate monitor users are exercising nearby. If that happens, the wristwatch may not accurately display the wearer's heart rate.

One type of analog chest strap sensor-transmitter transmits coded analog wireless signals. Coded analog transmission tend to reduce (but not eliminate entirely) the degree of cross talk while simultaneously preserving the ability to interface with remote heart rate monitor equipment.

A digital chest strap sensor-transmitter eliminates the problem of cross-talk when other heart rate monitor users are close by. By its very nature, the digital chest strap sensor transmitter is engineered to talk only to its own receiver (e.g., wristwatch).

Strapless heart rate monitors are wristwatch-type devices that may be preferred by users engaged in physical training because of convenience and combined time keeping features. In some cases the user is required to press a conductive contact on the face of the device to activate a pulse measurement sequence based on electrical sensing at the finger tip. However, this may require the user to interrupt physical activity, and does not always provide an "in-process" measurement and, therefore, may not be an accurate determination of heart rate during continuous exertion.

There are 2 sub-types of strapless heart rate monitors. The first type of monitor measures heart rate by detecting electrical impulses. Some wristwatch-type devices have electrodes on the device's underside in direct contact with the skin. These monitors are accurate (often called ECG or EKG accurate) but may be more costly. The second type of monitor measures heart rate by using optical sensors to detect pulses going through small blood vessels near the skin. These monitors based on optical sensors are less accurate than ECG type monitors but may be relatively less expensive.

Optical sensing, related to pulse oximetry, may also be used. The arrangement of heart rate sensor and display may be similar to that described above. The method of measurement is based on a backscattered intensity of light that illuminates the skin's surface and is sensitive to the change of red blood cell density beneath the skin during the pulse cycle. Motion of the sensor may introduce noise that corrupts the signal.

Compensation and removal of noise due to motion of an optical pulse sensor relative to the skin during exercise imposes an additional hardware and signal processing burden on the pulse monitoring device. An apparatus and method of signal processing that compensates and removes noise corrupting the actual pulse, and provides a user friendly apparatus (such as not requiring a chest or ankle sensor, or placement over an artery) would be beneficial and more convenient for physical training.

### SUMMARY

A heart rate monitor is disclosed comprising two main components. A first wristwatch type device measures three categories of sensor signal, digitizes the signals, correlates them to a generated clock signal, encodes them for transmission, and transmits the encoded data to a second device. An exemplary method of transmission may be Bluetooth, although other protocols may be employed, including hard wired signal transmission. The second device may be, for example, a smart phone (e.g., an iPhone™ or equivalent device equipped to transceiver wireless data) or other device, running an application to decode the transmitted data, process the signals to obtain a noise compensated heart rate, store data, and transmit a return signal to the first device on the basis of the processed signals. Additional data may be collected by the first device, such as battery life, pulse signal strength, and the like, which may also be transmitted to the second device. In turn, the second device may return signals to the first device to alert the user with status indicator, such as low battery, pulse rate too high/low, etc. More detailed information may be provided on the display of the second device.

In addition, audio data may be transmitted from the second device to audio earphones either coupled to the first device, or by further receiving a wireless signal such as via Bluetooth™.

### BRIEF DESCRIPTION OF THE DRAWINGS

FIG. 1 is a conceptual illustration of a heart rate sensing user system in accordance with the disclosure.
FIG. 2 is a conceptual illustration of a remote processing system for communicating with and controlling the user system of FIG. 1.
FIG. 3 is a conceptual illustration of a sensing system of the user system of FIG. 1.
FIG. 4 illustrates a conceptual view of the underside of the user system 100.
FIG. 5 illustrates a conceptual view of the front face of the user system 100.
FIG. 6 illustrates a method of operating a heart rate monitor comprising the heart rate sensing user system of FIG. 1 and the remote processing system of FIG. 2.

### DETAILED DESCRIPTION

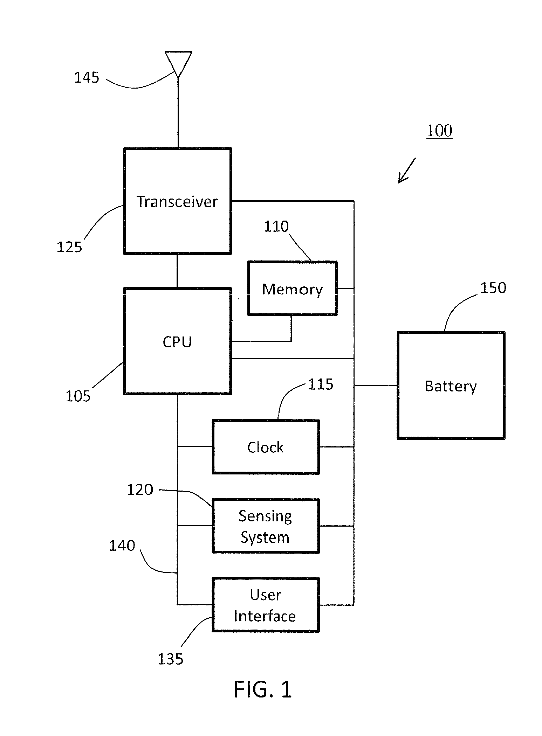

FIG. 1 illustrates a heart rate user system 100. The user system 100 may be worn on a user's wrist, but other locations besides the wrist, such as the ankle, arm or forearm may be used. The user system 100 includes a user processing CPU 105, a user memory 110, a clock signal generator 115, a sensing system 120, a user transceiver 125, and a user interface 135. The CPU 105 may be coupled to the other indicated components, for example, either directly or via a bus 140. A user antenna 145 is coupled to the user transceiver 125. The user antenna 145 may be a wireless connection to a remote processing system (discussed below), or it may be representative of a direct wired connection to the remote processing system.

A battery 150 is coupled to the user processor CPU 105, the user memory 110, the clock signal generator 115, the sensing system 120, the user transceiver 125, and the user interface 135 to power all functions of the indicated elements.

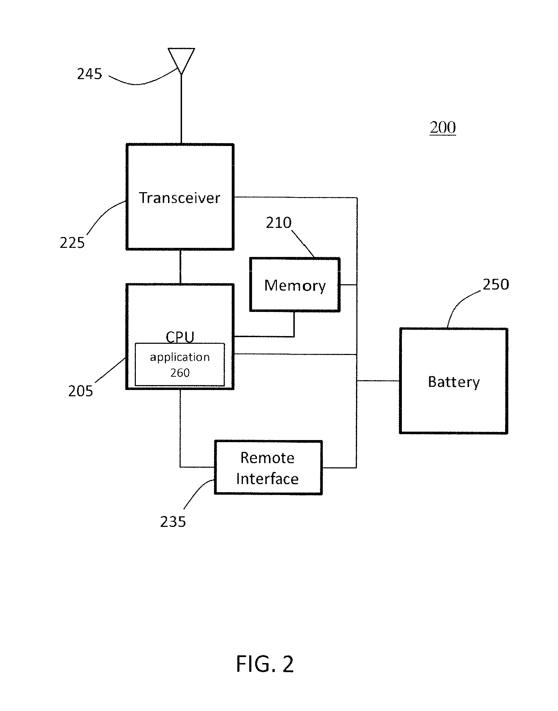

FIG. 2 illustrates a remote processing system 200 for receiving and analyzing signals transmitted from the user system 100. The remote processing system 200 may comprise, for example, a smart phone such as the Apple iPhone™ executing an application program to process the transmitted signals, as will be disclosed in more detail below. The remote processing system 200 may alternatively be a console, such as a dedicate piece of instrumentation for communicating and interacting with the user system 100. For example, the remote console may be used in a hospital, a fitness facility, or the like.

As an exemplary case, the remote processing system 200 may be a smart phone, such as an iPhone™, executing a heart rate monitoring application 260 on a remote CPU 205, where the application 260 may be stored in a remote memory 210 coupled to the remote CPU 205. The remote processing system 200 may also include a remote antenna 245 and a remote transceiver 225. The remote CPU 205 may execute the application commands and process the signals received from the user system 100, and generate output signals to the user system 100 via wireless transmission such as Bluetooth™, or the like, or via hard wire communication, on the basis of the processed received signals.

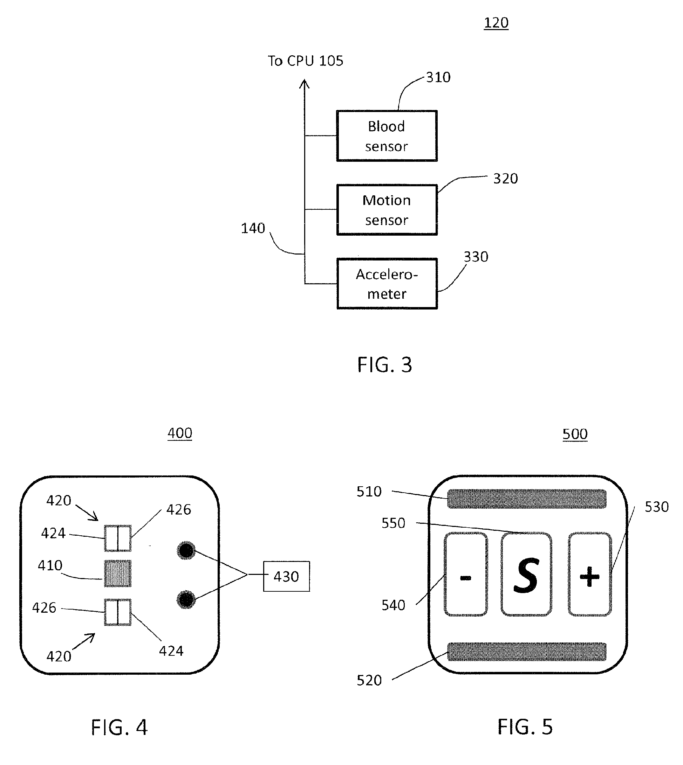

FIG. 3 is a conceptual illustration of the sensing system 120 of the user system 100 of FIG. 1. The sensing system 120 includes a blood concentration sensing system 310, described in more detail below. As the heart pumps blood through the arteries to microscopic blood vessels, the blood concentration varies periodically between a minimum and a maximum concentration, synchronously with a periodic variation in blood pressure. The blood concentration sensing system 310 senses this change in concentration in the blood vessels beneath the skin and transmits the signal level to the CPU 105. The blood concentration sensing system 310 may also sense small motions of the user system 100 with respect to the user's skin, because the blood concentration sensing system 310 may be sensing information from a different area of blood vessels beneath the user's skin if the user system 100 moves relative to the skin. This component of the sensed signal may be regarded as noise, which may contaminate a true determination of the heart rate.

Compensation of this signal is enabled by a sensor that is sensitive to motion, but not to blood concentration. The sensing system 120 further includes a motion sensor 320. The motion sensor functions in a manner analogous to a computer optical mouse, but is relatively insensitive to blood concentration near the surface of the skin. The motion sensor 320 senses changes in the position of the user system 100 with respect to the skin and sends a signal corresponding to that motion to the CPU 105, but contains relatively no substantial signal due to blood concentration. The signal from the motion sensor 320 and the signal from the blood concentration sensing system 310 may be correlated in time with the signal from the clock generator 115 to provide a compensated signal in which the noise contribution due to motion is substantially reduced. The compensated signal may then be analyzed for a more accurate determination of heart rate.

The sensing system further includes an accelerometer 330. The accelerometer 330 may be a chip-set comprising a plurality of sensing elements capable of resolving acceleration along three orthogonal axes. Microelectromechanical system (MEMS) sensors, capacitive sensors, and the like, are well known in the art of acceleration sensing. The accelerometer 330 may provide information about the motion of the user system 100 with respect to the user's heart. For example, if the user system 100 is worn on the wrist, and the nature of the exercise requires the wrists and hands to rise above the heart, the consequent elevation may cause a drop in the minimum and maximum (min/max) of the blood pressure at the point of sensing relative to that which may be measured when the user system 100 is as the same level or lower than the heart. This information may be used to qualify or disqualify the blood concentration measurements if the measured min/max values fall outside an acceptable range for determining the heart rate.

Some judgment may be used in making most effective use of the accelerometer 350. For example, if the exercise comprises bench presses, where the user's arms and hands are constantly being raised above the chest, placement of the user system at a relatively motion neutral location, such as an ankle or upper calf. The signal measured by the accelerometer 330 will not then indicate a shifting "baseline" for the effect of blood pressure on blood concentration measurements due to altitude change relative to the heart, and more data will qualify.

FIG. 4 illustrates a conceptual underside view 400 of the user system 100, showing elements of the blood concentration sensing system 310 and the motion sensor 320. In the illustration shown in FIG. 4, a photodetector 410 is positioned between two sets 420 of light emitting diodes (LEDs), although other light sources may be contemplated within the scope of the invention. Only one set 420 of LEDs is required, as a minimum, as discussed below, but a plurality of such LEDs can improve the sensitivity and performance of the user system 100. The photodetector 410 and the LED set 420 are positioned in close proximity, e.g., adjacent, to the user's skin and close to each other. Light emanating from an LED in the set 420 will penetrate a limited skin depth and a portion of the penetrating light will backscatter and be detected by the photodetector 310. As will be described below, the photodetector 410 has a spectral sensitivity that spans at least from green to red, or at least spanning the spectral bandwidths of the two LEDs.

For operation of the blood concentration sensing system 310, the LED set 420 includes a green LED 424. Green light is preferentially absorbed by red blood cells in the skin. Therefore, a systolic increase in blood pressure and vascular blood concentration during the course of a pulse may result in a decreased backscattered green light intensity. During the diastolic interval, blood concentration is lower, leading to an increased backscattered green light. The sensed signal level provided by the photodetector 410, when synchronized with the clock signal generator 115, may be analyzed under the control of a computer program stored in the user memory 110 and executable on the CPU 105 to determine a periodicity of the min/max signals, and thus determine a heart rate.

During exercise, a degree of motion of the user system 100 along the skin may occur. Because this changes the detailed microvascular network illuminated by the green LED 424, a motion signal, which may be regarded as noise, may be included in the backscattered green light. Therefore, a motion sensor independent of blood concentration is beneficial.

For operation of the motion sensor 320, the LED set 420 includes a red LED 426. Red light backscattered from vascular tissue in the skin is not substantially affected changes in blood concentration, and is not substantially sensitive to the pulsing of blood near the skin surface. However, the red LED 426 and photodetector 410 may function in a manner similar to an optical mouse, which is sensitive to motion relative to a surface, which in the present case happens to be the user's skin. The red LED 426 is used to sense small motion of the sensor with respect to the microvascular structure just beneath the skin. In an embodiment of the implementation of the motion sensor 320, the photodetector 410 may be a special purpose image processing chip that merasures pixel-to-pixel changes in light intensity to compute motion of the user system relative to the user's skin. Such motion is to be expected in the course of exercise. This may result in a variation in signal levels having a temporal spectrum consistent with the periodicity of physical motion and which corrupts the primary heart rate signal of interest.

Operation of both the blood concentration sensing system 310 and the motion sensor 320 with a common photodetector 410 is achieved by alternately firing the green LED 424 and the red LED 426 under control of the CPU 105, synchronized with the clock signal generator 115. Thus, the photodetector 410 must have sensitivity to spectral bands including both LED colors. The clock signal rate may be high enough, e.g., typically a kilohertz or more, that the two signals - for blood concentration and motion - may appear to be quasi-continuous, with enough granularity to extract sufficient detail from each - i.e., blood concentration and motion from the green LED 424 and motion only from the red LED 426.

One of the functions of the user CPU 105 may further include reading the battery level to the CPU 205 of the remote processing system 200 as transmitted, for example, via Bluetooth™, and returning a command to the user system 100 to display an indication that the battery level is normal or low.

Another function of the remote CPU 205 may be to determine, on the basis of the received sensor signals, whether the pulse signal peak values are too large (causing saturation) or two weak (causing poor signal-to-noise ratio (SNR)). If the detected pulse is two weak, the remote CPU 205 may provide feedback to the user CPU 105 instructing it increase the intensity of the LEDs by increasing the pulse peak power or pulse width, or reducing the intensity of the LEDs by reducing the pulse peak power or pulse width if the signal is saturating. Alternatively, the remote CPU 205 may provide feedback to the user CPU 105 instructing it to do the same. This is especially valuable because normative values of blood pressure may differ for different people, e.g., different skin color and light absorption properties, and may also change significantly as the course of a variable exercise regimen progresses through different levels of activity. For example, when the user is engaged in a sports activity, blood pressure and blood concentration is usually higher, so less light is required to pick up a signal. Therefore, the pulse driven fluctuation of the green LED light is affected by blood pressure, and the current to the green LED may be controlled to conserve power.

Alternatively, this function may be performed locally. In this alternative configuration, the user CPU 105 may be to determine, on the basis of the sensor signals output from the photodetector 410, or the power applied to the LEDs, whether the pulse signal peak values are too large (causing saturation) or too weak (causing poor signal-to-noise ratio (SNR)). If the detected pulse is too weak, the user CPU 205 may increase the pulse peak power or pulse width, or reduce the pulse peak power or pulse width if the signal is saturating. Alternatively, the user CPU 105 may increase gain in circuitry coupled to the photodetector 410 or reduce gain if the signal is saturating.

As described earlier, the operation of the blood concentration sensing system 310 and the motion sensor 320 with a common photodetector 410 may be achieved by alternately firing the green LED 424 and the red LED 426 under control of the CPU 105. In an alternative configuration of the sensing system, the firing sequence may include a blanking period after the green and red LEDs are fired. In this configuration, the user CPU 105 will cause, by way of example, the green LED 424 to fire, followed by the red LED 426, and then followed by a blanking period before the sequence repeats. The remote CPU 205 may then determine on the basis of the received sensor signal for the blanking period the effect that sunlight is having on the measurements. The remote CPU 205 may then compensate the received sensor signals for the green and red LEDs when computing the heart rate of the user, or provide this information back to the user CPU 105 in the form of feedback for adjusting the intensity of the green and red LEDS.

The user system 100 as shown in the underside view 400, may also include recharging ports 430 for recharging the user battery 150.

In one configuration of a user system 100, the user CPU 105 may be used to compute the user's heart rate based on information from the sensing system. The user CPU 105 may use information from the motion sensor and the accelerometer to compensate the information from the blood concentration sensing system when computing the heart rate. The CPU 105 may provide information to the user via the user interface based on the computed heart rate. By way of example, an indicator on the user interface may be enabled when the computed heart rate exceeds a maximum threshold set by the user.

Alternatively, the remote processing system 200 may be used to compute the heart rate based on information from the sensing system. The information from the sensing system may be provided to the remote system 200 by the user transceiver 125. The information may be received by the remote transceiver 225 in the remote system 200 and provided to the remote CPU 205 for processing. The remote CPU 205 may then compute the heart rate using information from the motion sensor and the accelerometer to compensate the information from the blood concentration sensing system.

Using the remote interface 235 of the remote processing system 200, an exercise schedule may be created. The remote interface 235 may be, for example, a touch screen, such as found on an APPLE iphone™, a smart phone keyboard and screen, and a screen, keyboard and mouse of a computer console. A maximum estimated heart rate may be determined based on various factors, including the user's age. A maximum estimated heart rate may correspond to an extreme level of performance, and different levels of performance may correspond to different ranges spanning from the maximum estimated heart rate down to a range corresponding to a resting state, so that a range of heart rates may be established for each range of exercise performance. Typical ranges of performance may correspond to resting, moderate exercise (e.g., walking), up to an extreme range corresponding to a maximum recommended level of activity, keeping in mind that such levels are only guidelines, and subject to appropriate modification. Having chosen a level of exercise, the remote processing system 200 CPU 205 may communicate via the transceivers 145 and 245 to the user system CPU 105 to signal when the received sensor signals indicate the heart rate is below, within, or above the selected exercise performance range. In this manner, the user may control and monitor his/her level of activity.

Referring now to FIG. 5, illustrating a conceptual view of the front face 500 of the user system 100, the user CPU 105, on the basis of performance range information received from the remote system 200, may control display features on the front face 500, away from the user's skin, which is thus accessible to the user. For example, in one embodiment, a red light indicator 510 on the display face may indicate that the heart rate is above a prescribed range for a selected exercise performance, and the user should exercise more slowly. Conversely, a green light indicator 520 may indicate that the performance level is below the prescribed range, and the user should exercise harder. At an appropriate level of exercise, neither light may be on, indicating an appropriate level of exercise is obtained. Other combinations of light indicators and colors may me contemplated within the scope of the invention.

Additional functionality may be included in the user system 100 in coordination with functionality available in the remote processing system 200. For example, the remote processing system 200 may also serve as an audio player (MP3, iPod™, etc.) storing a number of music tracks, or accessing a number of radio stations, made available by an appropriate entertainment software application running on the remote processing system 200 . Referring to FIG. 5, a set of buttons ("+" = volume up/track forward 530, "-" = volume down/track backward 540, and "select" S 550) on the user system 100 front face 500 enable the user to select an audio file or channel and volume. The select button S 550 may provide entertainment selection functions, such as pause, play, etc.

Additionally, the select button S 550 may serve as an emergency alert button. For example, repeated or continuously press S 550 may initiate a signal from the user system 100 to the remote processing system 200 to activate an alarm, such as an emergency alert phone message (911, private physician, or the like). If the remote processing system 200 is also equipped with GPS, the emergency alert message may also contain the location of the user, and vital statistics, such as the heart rate and/or high or low blood concentration level, which may indicate a high or low blood pressure, together with the identity of the user.

The remote system 200 may be carried by the user, for example, on a wrist, arm or waist strap, with viewing access easily available. The remote system 200 may therefore provide on its display (not shown) more detailed information, such as heart rate, calories burned, distance run, and the like, as determined by the application.

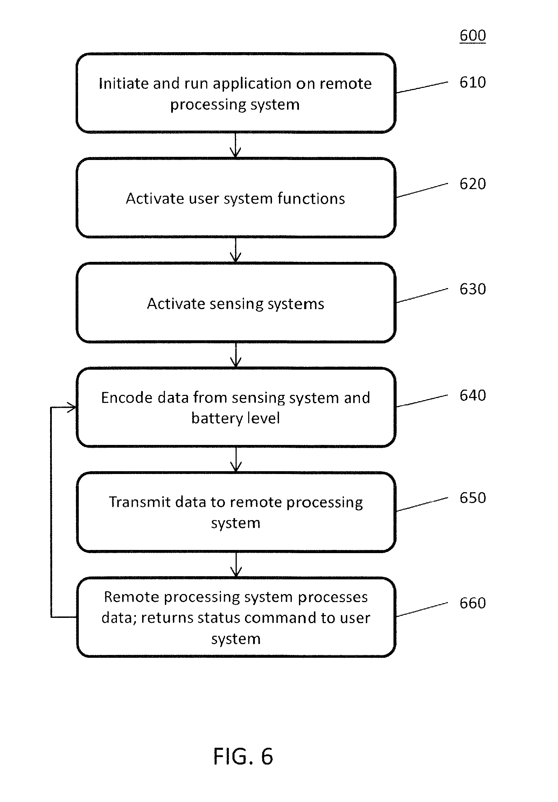

FIG. 6 illustrates a method 600 of operating the heart rate monitor comprising the user system 100 and the remote processing system 200. In block 610, the user initiates and runs the heart rate monitoring application 260 on the remote processing system 200. In block 620 the remote processing system 200 communicates with and activates the user system 100 heart monitor functions stored in the user memory 110 executable on the user CPU 105. The user system CPU 105 turns on operation routines controlling the sensing system 120 comprising the green LED 424, the red LED 426 and photodetector 410 and also the accelerometer operation routines in block 630. The routines control the operation of the LEDs, i.e., the repetition rate, alternating timing of the green and red LEDs, pulse widths of the LED output, and photodetector circuitry. The routines may also control the operation of the accelerometer 330 and associated circuitry. In block 640 the CPU 105 converts the analog signal from the photodetector , the accelerometer and the battery voltage to a digital signal that is then encoded for transmission as a data packet. In block 650, a signal is transmitted by the user system 100 CPU via the transceivers 225, 245, such as a Bluetooth™, and antennas 245, 345 to the remote processing system 200 including the blood concentration data, motion data accelerometer data, battery voltage, and clock signal. Alternatively, transmission may be via a hard wire link. In block 660, the remote processing system 200 CPU 205 processes the received data and may transmit various commands back to the user system 100 CPU 105. Among these include commands to turn on red or green LEDs on the front face of the user system to indicate to the user to exercise faster (green LED), exercise slower (red LED), and maintain the same level of exercise (no front LED lit).

The method functions continuously by returning, for example, to block 640, to obtain and encode the next packet of data.

The battery level may be indicated during charging. For example, when the user system is being charged through the charging ports 430, the green LED 510 may blink intermittently once for 25% charged, twice for 50% charged, three times for 75% charged, and steady on for 100% charged, or the like.

All operation conditions and exercise parameters may be visually presented on the user interface of the remote processing device 200, e.g., the touch screen of an iPhone™ or computer screen.

The remote processing device 200 display (not shown) may show a variety of data. Exemplary information that may be displayed include a numeric value of the measured (corrected) heart rate, a workout time indicator, a calorie counter, a level of performance indicator, exercise, pause and stop soft keys, and a music function soft key, all accessible using the multifunction key. Other functions may be contemplated as well.

It is to be understood that the specific order or hierarchy of steps in the methods disclosed is an illustration of exemplary processes. Based upon design preferences, it is understood that the specific order or hierarchy of steps in the methods may be rearranged. The accompanying method claims present elements of the various steps in a sample order, and are not meant to be limited to the specific order or hierarchy presented unless specifically recited therein.

The claims are not intended to be limited to the various aspects of this disclosure, but are to be accorded the full scope consistent with the language of the claims. All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112, sixth paragraph, unless the element is expressly recited using the phrase "means for" or, in the case of a method claim, the element is recited using the phrase "step for."

## Claims

1. A heart rate monitor configured to be worn against a user's skin, comprising:
a sensing system including:
a blood concentration sensing system configured to sense changes in blood concentration in one or more blood vessels beneath a surface of the skin,
a motion sensor configured to sense changes in position of the heart rate monitor with respect to the skin, and
an accelerometer configured to sense motion of the heart rate monitor with respect to a user's heart.

2. The heart rate monitor of claim 1 further comprising a processor configured to compute a user's heart rate based on information from the sensing system.

3. The heart rate monitor of claim 1 further comprising a processor configured to compute a user's heart rate using information from the motion sensor and the accelerometer to compensate information from the blood concentration sensing system.

4. The heart rate monitor of any one of claims 1 to 3 further comprising a transceiver configured to:
transmit information from the sensing system to a remote system for computing a user's heart rate, and
receive information related to the user's heart rate from the remote system for display to the user.

5. The heart rate monitor of any one of claims 1 to 4 wherein the blood concentration sensing system comprises one or more green LEDs arranged with the heart rate monitor such that at least a portion of light emitted from the one or more green LEDs penetrates the skin.

6. The heart rate monitor of claim 5 wherein the blood concentration sensing system further comprises a photodetector arranged with the heart rate monitor to detect backscatter from said at least a portion of the light that penetrates the skin.

7. The heart rate monitor of any one of claims 1 to 6 wherein the motion sensor comprises one or more red LEDs arranged with the heart rate monitor such that at least a portion of light emitted from the one or more red LEDs penetrates the skin.

8. The heart rate user monitor of claim 7 wherein the motion sensor further comprises a photodetector arranged with the heart rate monitor to detect backscatter from said at least a portion of the light that penetrates the skin.

9. The heart rate monitor of any one of claims 1 to 4 wherein the blood concentration sensing system further comprises one or more green LEDs and the motion sensor comprises one or more red LEDs, wherein the LEDs are arranged with the heart rate monitor such that at least a portion of green light emitted from the one or more green LEDs and at least a portion of red light emitted from the one or more red LEDs penetrate the skin.

10. The heart rate monitor of claim 9 further comprising a processor configured to:
enable the one or more green LEDs during a first time period and disable the one or more green LEDs during a second time period, and
disable the one or more red LEDs during the first time period and enable the one or more red LEDs during the second time period.

11. The heart rate monitor of claim 9 wherein the blood concentration sensing system and the motion sensor comprise a common photodetector, and wherein the photodetector is arranged with the heart rate monitor to detect backscatter from said at least a portion of the green light and said at least a portion that red light that penetrate the skin.

12. The heart rate monitor of claim 11 further comprising a processor configured to:
enable the one or more green LEDs during a first time period and disable the one or more green LEDs during a second time period,
disable the one or more red LEDs during the first time period and enable the one or more red LEDs during the second time period, and
disable the one or more green LEDs and the one or more red LEDs during a third time period.

13. The heart rate monitor of claim 12 wherein the processor is further configured to determine whether the photodetector saturates during the third time period and reduce sensitivity of the photodetector if the processor determines that the photodetector saturates during the third time period.

14. The heart rate monitor of claim 13 wherein the processor is further configured to increase power of the one or more green LEDs and the one or more red LEDs if the processor determines that the photodetector saturates during the third time period.

15. The heart rate monitor of claim 12 wherein the processor is further configured to compute a user's heart rate using information from the photodetector during the third time period to compensate information from the photodetector during the first and second time periods.

16. The heart rate monitor of claim 9 further comprising a processor configured to receive feedback related to power of the one or more green LEDs and the one or more red LEDs and adjust the power based on the feedback.
